# EUROPEAN PATENT SPECIFICATION

(11) **EP 4 042 935 B1**
(45) Date of publication and mention of the grant of the patent: **19.02.2025**
(21) Application number: 20888908.9
(22) Date of filing: 28.10.2020
(51) Int. Cl.: A61B 5/0205, A61B 5/024, A61B 5/0245, A61B 5/361, A61B 5/363, A61B 5/364, A61B 5/00

(54) **HEART STATE EVALUATION APPARATUS AND TERMINAL DEVICE**
VORRICHTUNG UND ENDGERÄT ZUR EVALUIERUNG DES HERZZUSTANDS
APPAREIL D'ÉVALUATION DE L'ÉTAT DU COEUR ET DISPOSITIF TERMINAL

(30) Priority: 22.11.2019 CN 201911155678
(43) Date of publication of application: 17.08.2022
(73) Proprietor: Huawei Technologies Co., Ltd., Shenzhen, Guangdong 518129 (CN)
(72) Inventor: GUO, Guangming, Shenzhen, Guangdong 518129 (CN); CHEN, Maolin, Shenzhen, Guangdong 518129 (CN); LI, Luping, Shenzhen, Guangdong 518129 (CN)
(74) Representative: MERH-IP Matias Erny Reichl Hoffmann Patentanwälte PartG mbB
(86) International application number: PCT/CN2020/124486
(87) International publication number: WO 2021/098461

(56) References cited:
- CN-A- 105 748 063
- CN-A- 106 725 428
- CN-A- 106 805 965
- CN-A- 108 403 107
- CN-A- 109 887 594
- CN-A- 110 037 681
- US-A- 6 108 578
- US-A1- 2014 257 122
- US-A1- 2017 238 814
- US-A1- 2019 059 763
- US-B1- 6 192 273

## Description

### TECHNICAL FIELD

This application relates to the field of data processing technologies, and in particular, to a heart status assessment apparatus and terminal device.

### BACKGROUND

Low and medium-risk arrhythmias are very common in today's society. For sporadic, asymptomatic arrhythmias, medication usually is not recommended by doctors. However, without intervention, the low and medium-risk arrhythmias may place a burden on hearts, which results in a frequent occurrence of arrhythmia diseases and an increasing number of young people suffering the arrhythmia diseases.

In the existing diagnosis and treatment solutions, data collected by a hospital, such as electronic medical records, medical images, and ICU monitor data, can be used to comprehensively analyze and predict risks of heart diseases for users. However, such diagnosis and treatment solutions mainly target patients in an advanced stage, focusing on high-risk diseases that pose a death threat. The low and medium-risk arrhythmias are not focused by the doctors, and therefore it is difficult for the doctors to intervene at an early reversible phase of arrhythmias by reminding the users of any potential risk. Furthermore, it is difficult to popularize such diagnosis and treatment solutions.

In addition, currently, many intelligent wearable devices have a heart rhythm detection function. However, these intelligent wearable devices can only display simple prompts such as "no abnormality", "sporadic irregular heart rhythm", and "suspected atrial fibrillation". Because most users lack knowledge about heart diseases, it is difficult for the users to understand these prompts. In addition, these prompts use a coarse-grained assessment scale. Therefore, the users can only learn whether heart rhythms are abnormal, and can hardly recognize heart health degrees.

In conclusion, because the existing heart health prompt solution is not intuitive with the coarse-grained assessment scale, it is hard for the users to recognize the heart health degrees.
Document US 2017/238814 A1 generally discloses a dashboard centered around arrhythmia or atrial fibrillation tracking. The dashboard includes a heart or cardiac health score that can be calculated in response to data from the user such as their ECG and other personal information and cardiac health influencing factors. The dashboard also provides to the user recommendations or goals, such as daily goals, for the user to meet and thereby improve their heart or cardiac health score. These goals and recommendations may be set by the user or a medical professional and routinely updated as his or her heart or cardiac health score improves or otherwise changes. The dashboard is generally displayed from an application provided on a smartphone or tablet computer of the user.
Document US 2019/059763 A1 generally discloses a method of detecting abnormalities in electrocardiogram (ECG) signals, the method comprising receiving a set of ECG signals from an ECG device; amplifying only the peaks of at least some of the set of ECG signals to produce ECG beat markings from which a heart rate is derivable to detect an irregular rhythm between at least two ECG beats; extracting a single ECG beat from the set of ECG signals from the ECG device by using said ECG beat markings; feeding the extracted single ECG beat into a first neural network; producing, at the first neural network, a compact representation of the extracted single ECG signal so as to generate a feature extraction output; and using, at a second neural network, the feature extraction output from the first neural network to generate a score associated with the abnormalities in the ECG signals.

### SUMMARY

In view of this, embodiments of this application provide a heart status assessment apparatus and terminal device to resolve a problem that it is difficult for a user to recognize a heart health degree because the existing heart health prompt solution is not intuitive with a coarse-grained assessment scale. In particular, embodiments of the present application are defined by the independent claims. Additional features of embodiments of the invention are presented in the dependent claims. In the following, parts of the description and drawings referring to former embodiments which do not necessarily comprise all features to implement embodiments of the claimed invention are not represented as embodiments of the invention but as examples useful for understanding the embodiments of the invention. Embodiments of the present application (or the present disclosure) provide a heart status assessment apparatus and a terminal.

A first aspect of embodiments of this application, not claimed as such but useful to understand the invention, provides a heart status assessment method, including
obtaining heart rhythm sample data, where the heart rhythm sample data includes sample heart rhythm features and sample labels;
training a preset scoring model based on the heart rhythm sample data, to obtain a heart rhythm scoring model; and
inputting a to-be-recognized heart rhythm feature into the heart rhythm scoring model, to obtain a heart status assessment score.

It should be noted that, the sample heart rhythm features may include one or more of features such as a time domain feature (for example, an RMSSD indicator, an NN50 indicator, and a PNN50 indicator), a frequency domain feature (for example, a total power, an ultra-low frequency power, an extremely low frequency power, a low frequency power, a high frequency power, and a ratio of low frequency to high frequency), an average, a variance, a maximum value, a minimum value of heart rates, an attack frequency, and attack duration.

The preset scoring model may be trained based on the sample heart rhythm features and the sample labels in the heart rhythm sample data, to obtain the heart rhythm scoring model. The heart rhythm scoring model may assess the to-be-recognized heart rhythm feature, to obtain the heart status assessment score.

A heart status of a user is displayed in a form of the heart status assessment score, so that the user can intuitively learn a current heart health degree of the user. In addition, the heart status is assessed by using the heart status assessment score, and an assessment granularity is fine, so that the user can accurately learn the current heart health degree of the user.

In a possible implementation of the first aspect, the sample labels are sample scores, and the preset scoring model is a regression model.

It should be noted that the sample labels may be the sample scores. The sample scores may be scores given by a person or a machine.

The preset scoring model may be the regression model. The regression model may be a linear regression model or a non-linear regression model. A specific type of the regression model may be selected based on an actual requirement.

In another possible implementation of the first aspect, the sample labels are ranks, and the preset scoring model is a ranking model.

It should be noted that the preset scoring model may be the ranking model. A type of the ranking model may be selected based on an actual requirement.

In a possible implementation of the first aspect, the obtaining heart rhythm sample data includes:
ranking all pieces of heart rhythm sample data based on a sample heart rhythm feature of each piece of heart rhythm sample data and a preset ranking rule, to obtain ranks of all the pieces of heart rhythm sample data.

It should be noted that the user may preset an appropriate ranking rule, and all the pieces of heart rhythm sample data are automatically ranked based on the sample heart rhythm feature of each piece of heart rhythm sample data and the preset ranking rule, to obtain the corresponding ranks of all the pieces of heart rhythm sample data.

All pieces of heart rhythm sample data can be automatically ranked by setting the appropriate ranking rule. Compared with the mode of manual scoring, using the ranks as the sample labels can reduce a lot of manpower and material resources.

In a possible implementation of the first aspect, the obtaining heart rhythm sample data includes:
obtaining original data, to obtain at least one data segment after performing window-based processing on the original data based on a preset time window; and
extracting a heart rhythm feature of the data segment, to obtain a sample heart rhythm feature.

It should be noted that the original data may be one piece of data, or may be a plurality of pieces of data. An appropriate time window is set, and at least one data segment can be obtained by performing window-based processing on the original data based on the preset time window.

Then, a feature extraction operation is performed on the data segment, to obtain the sample heart rhythm feature.

In a possible implementation of the first aspect, after the obtaining heart rhythm sample data, the method further includes:
determining a scaling algorithm based on the sample labels and a preset interval; and
correspondingly, the inputting a to-be-recognized heart rhythm feature into the heart rhythm scoring model, to obtain a heart status assessment score includes:
   inputting the to-be-recognized heart rhythm feature into the heart rhythm scoring model, to obtain a first assessment score;
   calculating the first assessment score by using the scaling algorithm, to obtain a second assessment score; and
   using the second assessment score as the heart status assessment score.

It should be noted that normalization processing may be performed on the heart status assessment score according to a proportion, so that the user can intuitively learn the heart status.

In a possible implementation of the first aspect, the using the second assessment score as the heart status assessment score includes:
if the second assessment score is within the preset interval, using the second assessment score as the heart status assessment score;
if the second assessment score is less than a lower limit of the preset interval, using the lower limit as the heart status assessment score; and
if the second assessment score is greater than an upper limit of the preset interval, using the upper limit as the heart status assessment score.

It should be noted that, through out-of-bounds correction, the heart status assessment score can be maintained in the preset interval, to prevent the user having unnecessary doubts when the user views the heart status assessment score.

In a possible implementation of the first aspect, the method further includes:
obtaining all heart status assessment scores that are within a preset time period; and
matching all the heart status assessment scores that are within the preset time period with heart score curves in a preset database, where a heart score curve with a highest matching degree is presented as a prediction curve.

It should be noted that, after the heart status assessment score is obtained, all the heart status assessment scores of the user within the preset time period are matched with the heart score curves in the database, where the heart score curve with the highest matching degree is used as the prediction curve. The prediction curve is obtained through big data matching, so that the user can intuitively learn a possible trend of future heart status changes.

In another possible implementation of the first aspect, the method further includes:
obtaining all heart status assessment scores that are within a preset time period; and
performing curve fitting on all the heart status assessment scores that are within the preset time period, to obtain and present a prediction curve.

It should be noted that, in addition to big data matching, curve fitting may be performed on the heart status assessment scores of the user within the preset time period, and the prediction curve is obtained through curve fitting, so that the user can conveniently and intuitively learn a possible trend of future heart status changes.

In a possible implementation of the first aspect, the method further includes:
performing, based on a prompt condition that the heart status assessment score meets, a preset prompt operation corresponding to the prompt condition.

It should be noted that, after the heart status assessment score is obtained, the preset prompt operation corresponding to the prompt condition may be further performed based on the prompt condition that the heart status assessment score meets, to give an alert to the user and provide a proper suggestion for the user.

A second aspect of embodiments of this application provides a heart status assessment apparatus, according to independent claim 1.

In a possible implementation of the second aspect, the sample labels are sample scores, and the preset scoring model is a regression model.

In a possible implementation of the second aspect, the sample obtaining module includes:
a ranking label submodule, configured to rank all the pieces of heart rhythm sample data based on a sample heart rhythm feature of each piece of heart rhythm sample data and a preset ranking rule, to obtain ranks of all the pieces of heart rhythm sample data.

In a possible implementation of the second aspect, the sample obtaining module includes:
a window-based processing submodule, configured to obtain original data, where window-based processing is performed on the original data based on a preset time window, to obtain at least one data segment; and
a feature extraction submodule, configured to extract a heart rhythm feature of the data segment, to obtain a sample heart rhythm feature.

In a possible implementation of the second aspect, the apparatus further includes:
a scaling selection module, configured to determine a scaling algorithm based on the sample labels and a correspondingly, the status assessment module includes:
   a first assessment submodule, configured to input the to-be-recognized heart rhythm feature into the heart rhythm scoring model, to obtain a first assessment score;
   a second assessment submodule, configured to calculate the first assessment score by using the scaling algorithm, to obtain a second assessment score; and
   a score assessment submodule, configured to use the second assessment score as the heart status assessment score.

In a possible implementation of the second aspect, the score assessment submodule includes:
an in-interval submodule, configured to use the second assessment score as the heart status assessment score if the second assessment score is within the preset interval;
a lower limit submodule, configured to use a lower limit as the heart status assessment score if the second assessment score is less than the lower limit of the preset interval; and
an upper limit submodule, configured to use an upper limit as the heart status assessment score if the second assessment score is greater than the upper limit of the preset interval.

In a possible implementation of the second aspect, the apparatus further includes:
a score obtaining module, configured to obtain all heart status assessment scores that are within a preset time period; and
a first prediction module, configured to match all the heart status assessment scores that are within the preset time period with heart score curves in a preset database, where a heart score curve with a highest matching degree is presented as a prediction curve.

In another possible implementation of the second aspect, the apparatus further includes:
a score obtaining module, configured to obtain all heart status assessment scores that are within a preset time period; and
a second prediction module, configured to perform curve fitting on all the heart status assessment scores that are within the preset time period, to obtain and present a prediction curve.

In a possible implementation of the second aspect, the apparatus further includes:
a prompt operation module, configured to perform, based on a prompt condition that the heart status assessment score meets, a preset prompt operation corresponding to the prompt condition.

A third aspect of embodiments of this application provides a terminal device according to independent claim 9.

A fourth aspect of embodiments of this application, not claimed as such but useful to understand the invention, provides a computer-readable storage medium, where the computer-readable storage medium stores a computer program. When the computer program is executed by a processor, the steps in the foregoing method are implemented.

A fifth aspect of embodiments of this application, not claimed as such but useful to understand the invention, provides a computer program product. When the
computer program product is run on a terminal device, the terminal device is enabled to implement the steps in the foregoing method.

Compared with the conventional technology, embodiments of this application have the following beneficial effects:
In the heart status assessment method provided in this application, a heart rhythm scoring model is trained based on heart rhythm sample data, and a to-be-recognized heart rhythm feature is input into the heart rhythm scoring model, to obtain a heart status assessment score. A heart status of a user is displayed in a form of the heart status assessment score, so that the user can intuitively learn a current heart health degree of the user. In addition, the heart status is assessed by using the heart status assessment score, and an assessment granularity is fine, so that the user can accurately learn the current heart health degree of the user. This resolves a problem that it is difficult for the user to recognize the heart health degree because the existing heart health prompt solution is not intuitive with a coarse-grained assessment scale.

### BRIEF DESCRIPTION OF DRAWINGS

To explain the technical solutions in embodiments of this application more clearly, the following briefly describes the accompanying drawings used for describing the embodiments or the conventional art. It is clear that the accompanying drawings in the following description show merely some embodiments of this application, and a person of ordinary skill in the art may still derive other drawings from these accompanying drawings without creative efforts.
FIG. 1 is a schematic flowchart of a heart status assessment method according to an embodiment of this application;
FIG. 2 is a schematic diagram of a sample label according to an embodiment of this application;
FIG. 3 is a schematic diagram of an application scenario according to an embodiment of this application;
FIG. 4 is a schematic diagram of another application scenario according to an embodiment of this application;
FIG. 5 is a schematic diagram of still another application scenario according to an embodiment of this application;
FIG. 6 is a schematic diagram of a structure of a heart status assessment apparatus according to an embodiment of this application; and
FIG. 7 is a schematic diagram of a terminal device according to an embodiment of this application.

### DESCRIPTION OF EMBODIMENTS

The invention is defined by the appended claims.

In the following description, to illustrate rather than limit, specific details such as a particular system structure, and a technology are provided to make a thorough understanding of embodiments of this application. However, a person skilled in the art should know that this application may also be implemented in other embodiments without these specific details. In other cases, detailed description of well-known systems, apparatuses, circuits, and methods are omitted, so that this application is described without being obscured by unnecessary details.

To describe the technical solutions of this application, the following uses specific embodiments for description.

It should be understood that, when used in this specification and the claims of this application, the term "including" indicates presence of described features, entireties, steps, operations, elements, and/or components, but does not exclude presence or addition of one or more other features, entireties, steps, operations, elements, components, and/or collections thereof.

It should be further understood that the terms used in this specification are merely for describing specific embodiments, and are not intended to limit this application. Terms "one", "a" and "the" of singular forms as used in this specification and the claims of this application are intended to include plural forms, unless otherwise specified in the context clearly.

It should be further understood that the term "and/or" used in the specification and the claims of this application indicates any combination and all possible combinations of one or more items listed in association, and includes the combinations.

As used in the specification and claims of this application, depending on the context, the term "if" may be explained as "while" or "when" or "in response to determining" or "in response to detection". Similarly, depending on the context, the phrases "if it is determined that" or "if it is detected that [a stated condition or event]" may be explained as "when it is determined that" or "in response to determining" or "when it is detected (a stated condition or event)" or "in response to detecting (a stated condition or event)".

In addition, in the description of this application, the terms "first", "second", "third", and the like are merely used for differentiation and description, and cannot be understood as an indication or implication of relative importance.

A data classification method provided in embodiments of this application may be applied to a terminal device such as a mobile phone, a tablet computer, a wearable device, a vehicle-mounted device, an augmented reality (augmented reality, AR) device/a virtual reality (virtual reality, VR) device, a notebook computer, an ultra-mobile personal computer (ultra-mobile personal computer, UMPC), a netbook, or a personal digital assistant (personal digital assistant, PDA). A specific type of the terminal device is not limited in embodiments of this application.

For example, the terminal device may be a station (STATION, ST) in a WLAN, and may be a cellular phone, a cordless phone, a session initiation protocol (Session Initiation Protocol, SIP) phone, a wireless local loop (Wireless Local Loop, WLL) station, a personal digital assistant (Personal Digital Assistant, PDA) device, a handheld device with a wireless communication function, a computing device or another processing device connected to a wireless modem, a vehicle-mounted device, an terminal in an Internet of Vehicles, a computer, a laptop computer, a handheld communications device, a handheld computing device, a satellite wireless device, a wireless modem card, a television set-top box (set top box, STB), customer premise equipment (customer premise equipment, CPE) and/or another device for communicating over a wireless system, and a terminal in a next-generation communications system, for example, a mobile terminal in a 5G network, or a mobile terminal in a future evolved public land mobile network (Public Land Mobile Network, PLMN).

Statistics have shown that many people have low and medium-risk arrhythmias. Cardiologists believe that if a sinus arrhythmia is taken into account, an incidence of an arrhythmia is 100%. If dynamic electrocardiograms are used to observe normal people for 24 hours, 70% to 80% of the people may have a symptom of premature contractions. If the electrocardiograms are used to monitor the general people for seven days, it may be found that about half of the people have arrhythmia symptoms such as premature ventricular contractions (premature ventricular contractions), premature atrial contractions (premature atrial contractions), atrial tachycardia (atrial tachycardia), and atrial fibrillation (atrial fibrillation). Therefore, it is common for people to have the arrhythmia symptoms. The difference only lies in onset ages, attack duration, types, associated symptoms, and the like of the arrhythmias.

Doctors usually do not recommend medication for sporadic and non-sustained symptoms of the low and medium-risk arrhythmias, such as supraventricular tachycardia (supraventricular tachycardia), atrial flutter (atrial flutter), atrial fibrillation (atrial fibrillation), and sinus arrhythmia. However, without appropriate intervention, the low and medium-risk arrhythmias can also place a burden on a heart, which results in a frequent occurrence of arrhythmia diseases and an increasing number of young people suffering the arrhythmia diseases.

Currently, heart diseases are becoming more prevalent and there is an increasing number of young people suffering the heart diseases. The heart diseases are the primary cause of death for cardiovascular patients. For the heart diseases, the best treatment solution is to apply appropriate intervention at a reversible phase of the disease when the disease carries a low and medium-risk.

In the existing diagnosis and treatment solutions, data collected by a hospital, such as electronic medical records, medical images, and ICU monitor data, can be used to comprehensively analyze and predict risks of the heart diseases for a user. However, such diagnosis and treatment solutions mainly target patients in an advanced stage, focusing on high-risk diseases that pose a death threat. The low and medium-risk arrhythmias are not focused by the doctors, and therefore it is difficult for the doctor to notice the user and to intervene at an early reversible phase of an arrhythmia. Furthermore, it is difficult to popularize such diagnosis and treatment solutions.

In addition, currently, many intelligent wearable devices have a heart rhythm detection function. However, a prompt solution of these intelligent wearable devices is simple. The devices can only simply display prompts such as "no abnormality", "sporadic irregular heart rhythm", and "suspected atrial fibrillation". Because most users lack knowledge about the heart diseases, it is difficult for the users to understand the prompt. In addition, an assessment granularity of the foregoing prompt solution is coarse. Therefore, the user can only simply learn whether a heart rhythm is abnormal, and it is hard for the user to recognize a heart health degree.

To resolve the foregoing problem, embodiments of this application provide a heart status assessment method and apparatus, a terminal device, and a storage medium. Details are as follows.

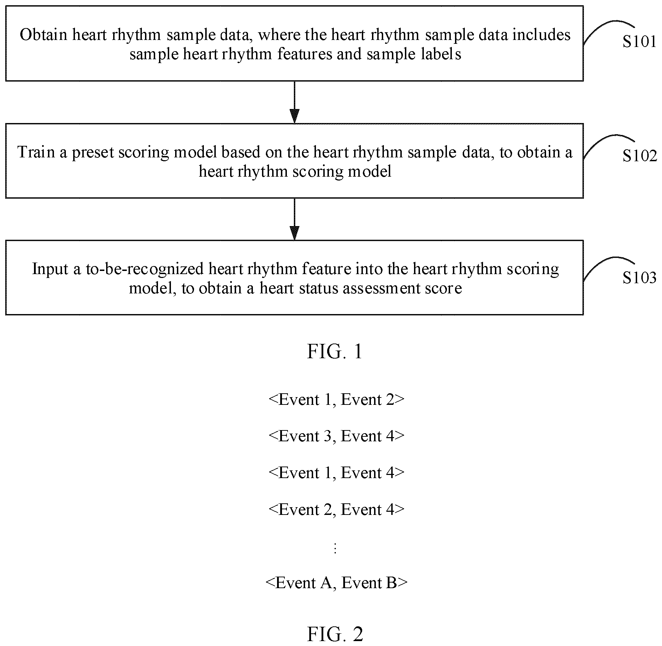

The following describes a heart status assessment method provided in an embodiment of this application. Refer to FIG. 1. The heart status assessment method in this embodiment of this application includes the following steps.

S101. Obtain heart rhythm sample data, where the heart rhythm sample data includes sample heart rhythm features and sample labels.

Before a heart status of a user is assessed, the heart rhythm sample data needs to be obtained first, and a heart rhythm scoring model is constructed based on the heart rhythm sample data.

The heart rhythm sample data may include the sample heart rhythm features and the sample labels. In an actual application, original data that can reflect a heart rhythm status of the user may be collected by using a sensor of a device such as a professional heart rhythm diagnosis and treatment device or an intelligent wearable device. A type of the original data may be determined based on an actual situation. For example, some devices may collect ECG (electrocardiography, electrocardiography) data, and some devices may collect PPG (photoplethysmography, photoplethysmography) data.

A time window of a preset time length is set, and the preset time length may be set based on an actual requirement. For example, the preset time length may be set to one minute, one hour, one day, or the like. The original data may be divided into several data segments by a time window.

After the several data segments are obtained, a heart rhythm feature in each data segment may be extracted. In a heart rhythm feature extraction process, a feature type to be extracted can be set based on an actual situation. For example, the extracted feature may include one or more of features such as a time domain feature (for example, an RMSSD indicator, an NN50 indicator, and a PNN50 indicator), a frequency domain feature (for example, a total power, an ultra-low frequency power, an extremely low frequency power, a low frequency power, a high frequency power, and a ratio of low frequency to high frequency), an average, a variance, a maximum value, a minimum value of heart rates, an attack frequency, and attack duration.

It may be understood that the foregoing data collection and feature extraction may be performed by a heart status assessment apparatus, or may be performed by another terminal device. For example, the existing intelligent wearable devices have a capability of collecting the ECG data/PPG data, and can perform feature extraction and identify a type of an arrhythmia event. Therefore, the intelligent wearable device may be used as the heart status assessment apparatus, and the intelligent wearable device performs data collection, feature extraction, and a subsequent processing procedure. Alternatively, another terminal device may be used as the heart status assessment apparatus. After performing data collection and feature extraction, the intelligent wearable device feeds back an operation result to the heart status assessment apparatus, and the heart status assessment apparatus performs the subsequent processing procedure.

After the heart rhythm features of the data segments are obtained, sample labels of the foregoing data segments need to be further obtained. In some possible implementations, if there is a small quantity of data segments, the data segments may be labeled manually, and sample scores obtained through manual assessment are used as the sample labels.

In a process of manual labeling, staff can score an arrhythmia event based on a severity level of the arrhythmia. In some embodiments, a scoring interval may be determined first based on an arrhythmia type. After scoring intervals corresponding to various types of arrhythmia events are determined, for arrhythmia events of a same type, severity levels of the arrhythmia events may be assessed based on factors such as attack duration, an amplitude of a deviation between a user heart rate and a normal heart rate, and a quantity of attacks, and scores of the arrhythmia events may be determined. A lower score indicates a more serious hazard posed by the arrhythmia event.

For example, after the several data segments are obtained, it may be first determined whether an arrhythmia event exists. If there is no abnormality, a score may be marked as 100. If there is an arrhythmia event, it is determined whether a type of the arrhythmia event can be identified. If the type of the arrhythmia event in the data segments cannot be identified, the data segments may be coarsely classified into three types: no obvious abnormality, abnormal heart rhythm, and suspected atrial fibrillation, and scores are 100, 50, and 0 respectively. If the type of the arrhythmia event can be identified, it is determined whether the arrhythmia event is a tachyarrhythmia type. If the arrhythmia event is not the tachyarrhythmia subtype, scoring is performed based on a deviation of a heart rate of the data segment relative to the normal heart rate, where the normal heart rate is a heart rate preset by the user. For example, the user may set a [60, 100] interval as an interval of the normal heart rate. For bradycardia (which usually refers to a heart rate lower than 60/min), a lower resting heart rate per minute indicates a lower score. For tachyarrhythmia (which usually refers to a heart rate higher than 100/min), a higher resting heart rate per minute indicates a lower score. If the arrhythmia event is the tachyarrhythmia type, the scoring interval is determined based on severity levels of tachyarrhythmia events of different types, and the severity levels of tachyarrhythmia are as follows: premature atrial contractions < persistent premature atrial contractions < atrial tachycardia < atrial flutter < atrial fibrillation. Then scoring intervals are determined respectively as: (100, 80], (80, 60], (60, 40], (40, 20], and (20, 0]. For tachyarrhythmia events of a same type, severity levels of the arrhythmia events may be assessed based on factors such as attack duration, an amplitude of a deviation between a user heart rate and a normal heart rate, and a quantity of attacks, and then scores of the arrhythmia events are determined. A lower score indicates a more serious hazard posed by the arrhythmia event.

In some other possible implementations, if there is a large quantity of data segments, time costs and labor costs of manual labeling are large. Alternatively, the data segments may be labeled in an automatic manner, to obtain the sample labels.

In an automatic labeling process, a series of ranking rules may be set, the data segments are ranked based on the severity levels of the arrhythmia events, and ranks (that is, ranking positions) are used as the sample labels. In some embodiments, ranking may be performed first based on different arrhythmia event types. For example, the severity levels of tachyarrhythmia are as follows: no abnormality < premature atrial contractions < persistent premature atrial contractions < atrial tachycardia < atrial flutter < atrial fibrillation. Ranks of the data segments of different arrhythmia event types may be determined based on this rule. For example, if a severity level of an arrhythmia event of the atrial fibrillation type is greater than a severity level of an arrhythmia event of the atrial tachycardia type, a data segment of the atrial fibrillation type is ranked before a data segment of the atrial tachycardia type, and a rank of the data segment of the atrial fibrillation type is less than a rank of the data segment of the atrial tachycardia type.

For the arrhythmia events of a same type, comparison may be performed based on preset indicators in the sample heart rhythm features. For example, the attack duration, the amplitude of a deviation between a user heart rate and a normal heart rate, and the quantity of attacks may be used as assessment indicators, and importance is as follows: attack duration > amplitude of a deviation between a user heart rate and a normal heart rate > quantity of attacks. The attack duration may be compared first. Longer attack duration indicates a higher severity level of an arrhythmia event and a lower rank. If the attack duration is the same, amplitudes of deviations between user heart rates and a normal heart rate may be compared. A larger deviation amplitude indicates a higher severity level of an arrhythmia event and a lower rank. If the deviation amplitudes are the same, quantities of attacks may be compared. A larger quantity of attacks indicates a higher severity level of an arrhythmia event and a lower rank.

In an actual application process, when the foregoing ranks are used as the sample labels, ranks corresponding to the data segments can be obtained only after the data segments are completely ranked and arranged. Consequently, a calculation workload is heavy, and system performance deteriorates. Therefore, in some other embodiments, ranks of different arrhythmia events may be used as the sample labels.

FIG. 2 is used as an example. A sample label may be represented in a form of <Event A, Event B>. The <Event A, Event B> indicates that a rank of Event A is smaller than that of Event B, and a severity level of Event A is greater than that of Event B. Directly using the ranks of different arrhythmia events as the sample labels can reduce the burden on system performance.

Automatic labeling of the data segments based on a ranking rule preset by the user is simple to implement. Massive data can be conveniently and automatically processed, which saves a large amount of manpower and material resources.

In addition to the two labeling manners of manual labeling performed by staff and automatic labeling, users with arrhythmias may also be invited to participate in labeling. The user may monitor and record heart rhythm data of the user, such as the ECG data and the PPG data, in real time by wearing a smart wristband. A degree of data abnormality is analyzed by using a preset algorithm (the heart rhythm features are analyzed as described above). When an abnormal heart rhythm is detected, the user is prompted to mark his/her feelings. The user may describe degrees of discomfort as different levels, for example, slight uncomfortable, moderate discomfort, or severe discomfort. Alternatively, the user may be invited to score, where a scoring interval may be set to [0, 100] and a higher score indicates a lower degree of discomfort. The arrhythmia events can be statistically ranked and scored through labeling performed by a large number of users having arrhythmias.

S102. Train a preset scoring model based on the heart rhythm sample data, to obtain a heart rhythm scoring model.

After the heart rhythm sample data is obtained, the preset scoring model may be trained based on the heart rhythm sample data.

In some possible implementations, a regression model may be selected as the preset scoring model. The regression model can be a linear regression model or a non-linear regression model. A specific type of the regression model may be selected based on an actual requirement. For example, one of regression models such as a linear regression (linear regression) model, a CART (Classification and regression tree, classification and regression tree) model, and a GBDT (Gradient Boosting Decision Tree, gradient boosting decision tree) model may be selected as the preset scoring model.

After the type of the regression model is determined, the regression model may be trained by using the sample heart rhythm features and the sample labels in the heart rhythm sample data. During iteration of training the regression model, an update algorithm may be selected based on an actual requirement. For example, a stochastic gradient descent method may be selected as the update algorithm of the regression model.

After training of the regression model is completed, a finally obtained regression model may be used as the heart rhythm scoring model.

In some other possible implementations, a ranking model may be selected as the preset scoring model. A type of the ranking model may be selected based on an actual requirement. For example, one of ranking models such as a Ranking SVM (ranking support vector machine) model, a GB Rank (ranking based on the gradient boosting decision tree) model, or a ListNet (ranking based on Listwise) model may be selected as the preset scoring model.

After the type of the ranking model is determined, the ranking model may be trained by using the sample heart rhythm features and the sample labels in the heart rhythm sample data. During iteration of training the ranking model, an appropriate update algorithm may be selected based on the type of the ranking model. For example, the GB Rank model may use a GBDT algorithm as the update algorithm, and the ListNet model may use a gradient descent (Gradient Descent) algorithm as the update algorithm.

After training of the ranking model is completed, a finally obtained ranking model may be used as the heart rhythm scoring model.

It should be understood that the regression model and the ranking model are merely examples in this embodiment of this application. In an actual application, another type of model may be selected as the heart rhythm scoring model based on an actual situation. The foregoing examples should not be understood as limitations on the type of the heart rhythm scoring model.

S103. Input a to-be-recognized heart rhythm feature into the heart rhythm scoring model, to obtain a heart status assessment score.

After the heart rhythm scoring model is obtained through training, a heart status may be assessed for the user by using the heart rhythm scoring model.

The user may collect to-be-recognized heart rhythm data that can reflect a heart rhythm status of the user, such as the ECG data or the PPG data, by using a sensor of a device, for example, a professional heart rhythm diagnosis and treatment device or an intelligent wearable device. The heart rhythm feature of the to-be-recognized heart rhythm data is extracted, and the to-be-recognized heart rhythm feature is input into the heart rhythm scoring model. The heart rhythm scoring model outputs a heart status assessment score.

In this embodiment of this application, the heart status of the user is displayed in the form of the heart status assessment score, so that the user can intuitively learn a current heart health degree of the user. In addition, the heart status is assessed by using the heart status assessment score, and an assessment granularity is fine, so that the user can accurately learn a current heart health degree of the user. This resolves a problem that it is difficult for the user to recognize how healthy a heart is because the existing heart health prompt solution is not intuitive with a coarse-grained assessment scale.

In some possible implementations, the sample scores or the ranks corresponding to the heart rhythm sample data may be scaled to a preset interval based on a preset scaling algorithm. The preset interval may be set based on a situation. For example, the preset interval may be set to an interval of [0, 100].

After the user obtains the heart status assessment score corresponding to the to-be-recognized heart rhythm feature by using the heart rhythm scoring model, the heart status assessment score may be used as a first assessment score. Then, scaling processing is performed on a second assessment score by using the same preset scaling algorithm, to obtain the second assessment score, and the second assessment score may be used as a final heart status assessment score.

The sample scores or the ranks of the heart rhythm sample data is scaled to the preset interval, and in a same manner, the assessment score output by the heart rhythm scoring model is scaled.

In the process of training the heart rhythm scoring model, the sample scores or the ranks of the selected heart rhythm sample data are not necessarily in a fixed interval. Therefore, when the user obtains the heart status assessment score, it is difficult to compare the heart status assessment score with a fixed interval, and a current heart state cannot be appropriately assessed.

For example, during the training of the heart rhythm scoring model, 10,000 pieces of heart rhythm sample data may be selected. When the sample labels are the ranks, sample labels of the 10,000 pieces of heart rhythm sample data are 1 to 10000. If an interval is not set and the sample labels are not scaled to the interval, the current heart state may not be appropriately assessed by the user based on the heart status assessment score. For example, the heart status assessment score obtained by the user is 2984. The user cannot directly determine whether the score 2984 is high or low, and therefore the user cannot determine whether the heart is in a healthy state. If the preset interval is set to [0, 100], if it is determined, based on the sample labels and the interval, that the heart status assessment score is reduced by 100 times, 2984 may be divided by 100 to obtain 29.84, and the heart status assessment score is displayed to the user in a 100-point system. After the user learns that the heart status assessment score is 29.84, the user can determine that 29.84 is at a low level in the [0, 100] interval, so that the user can intuitively learn that the current heart state is poor and medical treatment is needed in a timely manner.

Therefore, if the sample scores or the ranks of the heart rhythm sample data are scaled to the preset interval, and the assessment score output by the heart rhythm scoring model is scaled in the same manner, the user can conveniently compare the heart status assessment score with the interval, and intuitively learns the heart status.

In addition, when the heart status assessment score exceeds an upper limit and a lower limit of the preset interval, a specific value of the heart status assessment score is meaningless. For example, it is assumed that the preset interval is [0, 100], where 0 indicates that the to-be-assessed heart rhythm feature is a feature of a very severe arrhythmia event, and that the user is recommended to seek medical treatment in a timely manner; and 100 indicates that no abnormality is detected in the to-be-assessed heart rhythm feature. In this case, if the heart status assessment score is less than 0, whether the value is -10, -100, or -1000, the value indicates no other meanings. The user should seek medical treatment in a timely manner. If the heart status assessment score is greater than 100, whether the value is 200, 500, or 1000, the value indicates no other meanings, and only indicates that the user's heart is healthy and no intervention is needed by the heart.

In this case, a heart state that exceeds the upper limit and the lower limit of the preset interval affects measuring of the heart status by the user. Therefore, in some possible implementations, when the foregoing heart status assessment score can be further processed, it is determined whether the heart status assessment score is greater than the upper limit of the preset interval or less than the lower limit of the preset interval. If the heart status assessment score is greater than the upper limit, the heart status assessment score is the upper limit. If the heart status assessment score is less than the lower limit, the heart status assessment score is the lower limit. Through out-of-bounds correction, the heart status assessment score can be maintained in the preset interval, to prevent the user having unnecessary doubts when the user views the heart status assessment score.

For example, it is assumed that the preset interval is [0, 100], where 0 indicates that the to-be-assessed heart rhythm feature is a feature of a very severe arrhythmia event, and 100 indicates that no abnormality is detected in the to-be-assessed heart rhythm feature. In this case, it is assumed the heart status assessment score of the user is - 34. The user may wonder whether -34 indicates that the arrhythmia event is so severe that the arrhythmia cannot be treated, given that 0 indicates a very severe arrhythmia event. Therefore, if the heart status assessment score is less than 0, whether the value is -10, -100, or -1000, the value indicates no other meanings, and the user should seek medical treatment in a timely manner. If the heart status assessment score is directly displayed without being processed, on the contrary, the user will be more troubled. Alternatively, it is assumed that the heart status assessment score of the user is 145. The user may wonder what 145 indicates, given that 100 indicates that no abnormality is detected in the to-be-assessed heart rhythm feature, and whether 145 indicates that the heart status is very good or that the heart status is excessively good and abnormal. Therefore, if the heart status assessment score is greater than 100, whether the value is 200, 500, or 1000, no other meanings are indicated. The value only indicates that the user's heart is healthy, and no intervention is needed by the heart. If the heart status assessment score is directly displayed without being processed, on the contrary, the user may have more doubts. In this case, out-of-bounds correction may be performed on the heart status assessment score. If the heart status assessment score is greater than the upper limit, the heart status assessment score is the upper limit. If the heart status assessment score is less than the lower limit, the heart status assessment score is the lower limit. If the heart status assessment score of the user is -34, because -34 is less than 0, the heart status assessment score of the user may be set to 0. If the heart status assessment score of the user is 145, because 145 is greater than 100, the heart status assessment score of the user may be set to 100. When the user views the heart status assessment score obtained through out-of-bounds correction, the user can accurately measure the heart status of the user based on the preset interval. In this way, unnecessary doubts are prevented.

In some possible implementations, all heart status assessment scores of the user within a preset time period may be further obtained. All the heart status assessment scores of the user within the preset time period are matched with heart score curves recorded in a preset database, and a heart score curve with a highest matching degree is obtained. The heart score curves recorded in the preset database are historically collected change curves of heart status assessment scores of users.

A higher degree of matching between the heart status assessment scores of the user within the preset time period and the heart score curve indicates that the heart status assessment scores of the user within the preset time period and a trend of heart status assessment score changes are more similar to those of the heart score curve. Therefore, a trend of future heart status changes of the user may be predicted to some extent by using the heart score curve with the highest matching degree. The heart score curve is presented to the user, so that the user learns a possible trend of heart status changes of the user, and a corresponding health prompt is provided for the user.

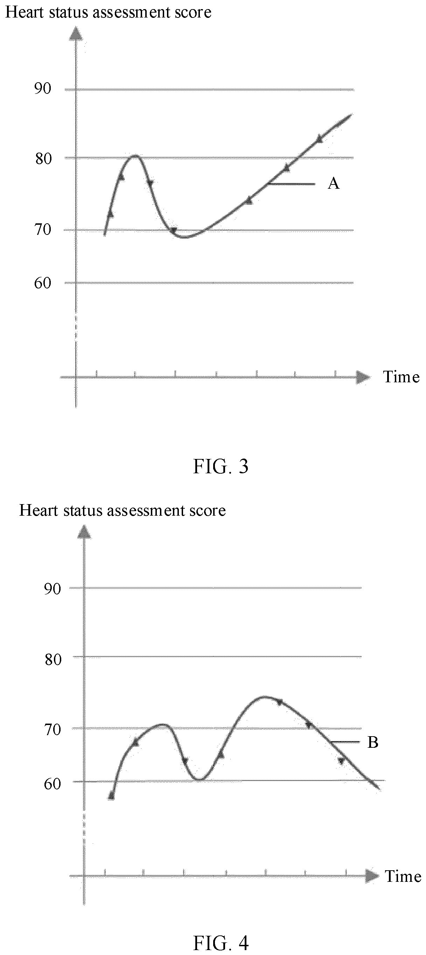

FIG. 3 to FIG. 5 are used as examples. Triangular data points in FIG. 3 are heart status assessment scores of user 1 within a preset time period. In the application scenario shown in FIG. 3, the heart status assessment scores of the user 1 are matched with the heart score curves stored in a preset database. A heart score curve A has a highest degree of matching to the heart status assessment scores of the user 1 and a change curve of the heart status assessment scores. The heart score curve A may be used to predict a trend of future heart status changes of the user 1.

A trend of the heart score curve A indicates that a latest heart status assessment score obtained by the user 1 is 83, a heart status of the user 1 within a future time period may be optimized, and the heart status assessment score may increase.

Triangular data points in FIG. 4 are heart status assessment scores of user 2 within a preset time period. In the application scenario shown in FIG. 4, the heart status assessment scores of the user 2 are matched with the heart score curves stored in the preset database.

A heart score curve B has a highest degree of matching to the heart status assessment scores of the user 2 and a change curve of the heart status assessment scores. The heart score curve B may be used to predict a trend of future heart status changes of the user 2.

A trend of the heart score curve B indicates that a latest heart status assessment score obtained by the user 2 is 64, and there is an arrhythmia event. If the user 2 does not seek medical treatment or perform intervention in time, the heart status of the user 2 may continuously deteriorate in a future time period, and the heart status assessment scores may continue to decline. In this case, the user may be prompted to seek medical treatment in time, or corresponding guidance may be provided based on a type of the arrhythmia event of the user.

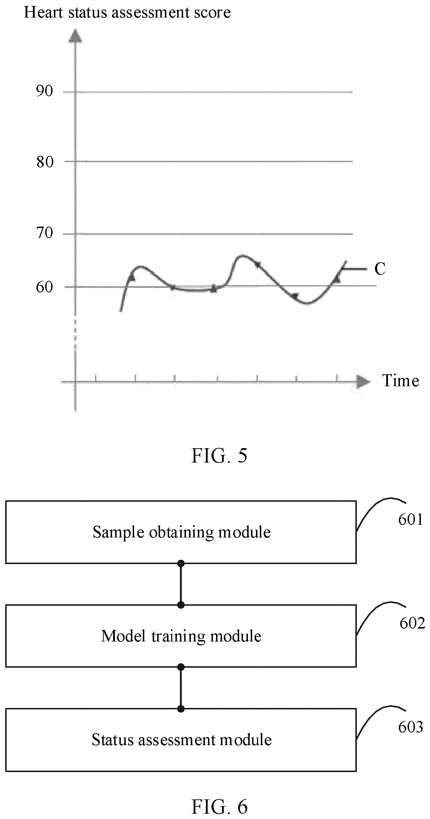

Triangular data points in FIG. 5 are heart status assessment scores of user 3 within a preset time period. In the application scenario shown in FIG. 5, the heart status assessment scores of the user 3 are matched with the heart score curves stored in the preset database.

A heart score curve C has a highest degree of matching to the heart status assessment scores of the user 2 and a change curve of the heart status assessment scores. The heart score curve C may be used to predict a trend of future heart status changes of the user 3.

A trend of the heart score curve C indicates that a latest heart status assessment score obtained by the user 3 is 61, and there is an arrhythmia event. However, the heart status of the user 3 may be optimized in a future time period, and the heart status assessment score may increase.

Therefore, all heart status assessment scores of the user within the preset time period are matched with the heart score curves recorded in the preset database, to obtain the heart score curve with the highest matching degree. The trend of future heart status changes of the user may be predicted to some extent by using the heart score curve. This helps the user learn the possible trend of heart status changes of the user, and also helps provide a corresponding health prompt for the user.

In some other possible implementations, curve fitting may be directly performed on heart status assessment scores of the user within a preset time period, to obtain a prediction curve and present the prediction curve to the user. A possible change of the heart status of the user in a future time period is predicted by using the prediction curve.

In some possible implementations, a corresponding preset prompt operation may be further performed based on the heart status assessment score. Content of the preset prompt operation may be set based on an actual situation. For example, if a currently obtained heart status assessment score falls within an interval from 80 to 100, the user may be prompted, by a voice prompt, that "A heart status is good". If the currently obtained heart status assessment score is less than 80 but higher than or equal to 40, the user may be notified of corresponding guidance by using a text, a picture, audio, or the like based on a type of a detected arrhythmia event, so that the user performs appropriate intervention according to the guidance to maintain heart health. If the obtained heart status assessment score is less than 40, an alert may be given to the user, so as to prompt the user to seek medical treatment as early as possible, and a contact information and an address of a nearby hospital are provided to the user.

It should be understood that sequence numbers of the steps do not mean an execution sequence in the foregoing embodiments. The execution sequence of the processes should be determined based on functions and internal logic of the processes, and should not constitute any limitation on the implementation processes of embodiments of this application.

Refer to FIG. 6. An embodiment of this application provides a heart status assessment apparatus. For ease of description, only parts related to this application are shown. As shown in FIG. 2, the heart status assessment apparatus includes:
a sample obtaining module 601, configured to obtain heart rhythm sample data, where the heart rhythm sample data includes sample heart rhythm features and sample labels;
a model training module 602, configured to train a preset scoring model based on the heart rhythm sample data, to obtain a heart rhythm scoring model; and
a status assessment module 603, configured to input a to-be-recognized heart rhythm feature into the heart rhythm scoring model, to obtain a heart status assessment score.

In a possible implementation of the second aspect, the sample labels are sample scores, and the preset scoring model is a regression model.

In another possible implementation of the second aspect, the sample labels are ranks, and the preset scoring model is a ranking model.

In a possible implementation of the second aspect, the sample obtaining module 601 includes:
a ranking label submodule, configured to rank all the pieces of heart rhythm sample data based on a sample heart rhythm feature of each piece of heart rhythm sample data and a preset ranking rule, to obtain ranks of all the pieces of heart rhythm sample data.

In a possible implementation of the second aspect, the sample obtaining module 601 includes:
a window-based processing submodule, configured to obtain original data and perform window-based processing on the original data based on a preset time window, to obtain at least one data segment; and
a feature extraction submodule, configured to extract a heart rhythm feature of the data segment, to obtain a sample heart rhythm feature.

In a possible implementation of the second aspect, the apparatus further includes:
a scaling selection module, configured to determine a scaling algorithm based on the sample labels and a preset interval; and
correspondingly, the status assessment module 603 includes:
   a first assessment submodule, configured to input the to-be-recognized heart rhythm feature into the heart rhythm scoring model, to obtain a first assessment score;
   a second assessment submodule, configured to calculate the first assessment score by using the scaling algorithm, to obtain a second assessment score; and
   a score assessment submodule, configured to use the second assessment score as the heart status assessment score.

In a possible implementation of the second aspect, the score assessment submodule includes:
an in-interval submodule, configured to use the second assessment score as the heart status assessment score if the second assessment score is within the preset interval;
a lower limit submodule, configured to use a lower limit as the heart status assessment score if the second assessment score is less than the lower limit of the preset interval; and
an upper limit submodule, configured to use an upper limit as the heart status assessment score if the second assessment score is greater than the upper limit of the preset interval.

In a possible implementation of the second aspect, the apparatus further includes:
a score obtaining module, configured to obtain all heart status assessment scores that are within a preset time period; and
a first prediction module, configured to match all the heart status assessment scores that are within the preset time period with heart score curves in a preset database, where a heart score curve with a highest matching degree is presented as a prediction curve.

In a possible implementation of the second aspect, the apparatus further includes:
a score obtaining module, configured to obtain all the heart status assessment scores that are within a preset time period; and
a second prediction module, configured to perform curve fitting on all the heart status assessment scores that are within the preset time period, to obtain and present a prediction curve.

In a possible implementation of the second aspect, the apparatus further includes:
a prompt operation module, configured to perform, based on a prompt condition that the heart status assessment score meets, a preset prompt operation corresponding to the prompt condition.

It should be noted that content such as information exchange and an execution process between the foregoing apparatuses/units is based on a same concept as that in the method embodiments of this application. For specific functions and technical effects of the content, refer to the method embodiments. Details are not described herein again.

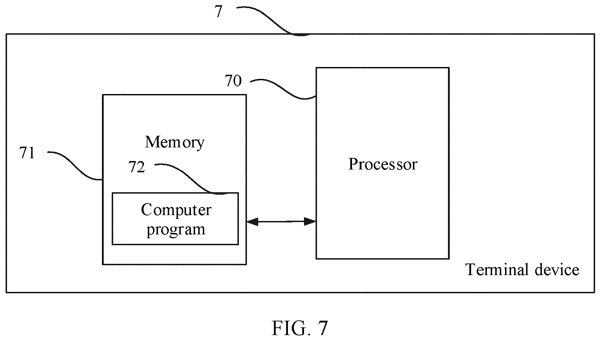

FIG. 7 is a schematic diagram of a terminal device according to Embodiment 3 of this application. As shown in FIG. 7, the terminal device 7 in this embodiment includes: a processor 70, a memory 71, and a computer program 72 that is stored in the memory 71 and that can run on the processor 70. When executing the computer program 72, the processor 70 implements the steps in the foregoing heart status assessment method embodiment, for example, S101 to S103 shown in FIG. 1. Alternatively, when executing the computer program 72, the processor 70 implements functions of the modules/units in the foregoing apparatus embodiment, for example, functions of the modules 601 to 603 shown in FIG. 6.

For example, the computer program 72 may be divided into one or more modules/units. The one or more modules/units are stored in the memory 71, and are executed by the processor 70, to complete this application. The one or more modules/units may be a series of computer program instruction segments that can implement a specific function, and the instruction segment is used to describe an execution process of the computer program 72 in the terminal device 7. For example, the computer program 72 may be divided into a sample obtaining module, a model training module, and a status assessment module. Specific functions of the modules are as follows.

The sample obtaining module is configured to obtain heart rhythm sample data, where the heart rhythm sample data includes sample heart rhythm features and sample labels.

The model training module is configured to train a preset scoring model based on the heart rhythm sample data, to obtain a heart rhythm scoring model.

The status assessment module is configured to input a to-be-recognized heart rhythm feature into the heart rhythm scoring model, to obtain a heart status assessment score.

The terminal device 7 may be a computing device such as a desktop computer, a notebook computer, a palmtop computer, or a cloud server. The terminal device may include, but is not limited to, the processor 70 and the memory 71. A person skilled in the art may understand that FIG. 7 is merely an example of the terminal device 7, and does not constitute a limitation on the terminal device 7. The terminal device may include more or fewer components than those shown in the figure, or some components may be combined, or different components may be used. For example, the terminal device may further include an input/output device, a network access device, a bus, or the like.

The processor 70 may be a central processing unit (Central Processing Unit, CPU), or may be another general-purpose processor, a digital signal processor (Digital Signal Processor, DSP), an application-specific integrated circuit (Application Specific Integrated Circuit, ASIC), a field-programmable gate array (Field-Programmable Gate Array, FPGA) or another programmable logic device, a discrete gate or a transistor logic device, a discrete hardware component, or the like. The general-purpose processor may be a microprocessor or the processor may be any conventional processor, or the like.

The memory 71 may be an internal storage unit of the terminal device 7, for example, a hard disk drive or an internal storage of the terminal device 7. The memory 71 may alternatively be an external storage device of the terminal device 7, for example, a removable hard disk, a smart media card (Smart Media Card, SMC), a secure digital (Secure Digital, SD) card, a flash card (Flash Card), or the like that is provided on the terminal device 7. Further, the memory 71 may alternatively include both the internal storage unit and the external storage device of the terminal device 7. The memory 71 is configured to store the computer program and other programs and data required by the terminal device. The memory 71 may further be configured to temporarily store data that has been output or is to be output.

It is clearly understood by a person skilled in the art that, for the purpose of convenient and brief description, division of the foregoing functional units and modules is taken as an example for illustration. In actual application, the foregoing functions can be allocated to different functional units and modules and implemented according to a requirement, that is, an inner structure of the apparatus is divided into different functional units and modules to implement all or some of the functions described above. The functional units and modules in the embodiments may be integrated into one processing unit, or each of the units may exist alone physically, or two or more units are integrated into one unit. The integrated unit may be implemented in a form of hardware, or may be implemented in a form of a software functional unit. In addition, specific names of the functional units and modules are merely used for distinguishing between each other, but are not intended to limit the protection scope of this application. For specific operating processes of the units and modules in the foregoing system, refer to the corresponding processes in the foregoing method embodiment, and details are not described herein again.

In the foregoing embodiments, the description of each embodiment has respective focuses. For a part that is not described in detail in an embodiment, refer to related description in other embodiments.

A person of ordinary skill in the art may be aware that, in combination with the examples described in embodiments disclosed in this specification, the units and algorithm steps may be implemented by electronic hardware or a combination of computer software and electronic hardware. Whether the functions are performed by hardware or software depends on particular applications and design constraints of the technical solutions. A person skilled in the art may use different methods to implement the described functions for each particular application, but it should not be considered that the implementation goes beyond the scope of this application.

In embodiments provided in this application, it should be understood that the disclosed apparatus/terminal device and method may be implemented in other manners. For example, the described apparatus/terminal device embodiment is merely examples. For example, division into the modules or units is merely logical function division and may be other division in actual implementation. For example, a plurality of units or components may be combined or integrated into another system, or some features may be ignored or not performed. In addition, the displayed or discussed mutual couplings or direct couplings or communication connections may be implemented through some interfaces. The indirect couplings or communication connections between the apparatuses or units may be implemented in electrical, mechanical, or other forms.

The units described as separate parts may or may not be physically separate, and parts displayed as units may or may not be physical units, may be located in one position, or may be distributed on a plurality of network units. Some or all of the units may be selected based on an actual requirement to achieve the objectives of the solutions of the embodiments.

In addition, the functional units in embodiments of this application may be integrated into one processing unit, or each of the units may exist alone physically, or two or more units are integrated into one unit. The integrated unit may be implemented in a form of hardware, or may be implemented in a form of a software functional unit.

When the integrated module/unit is implemented in a form of a software functional unit and sold or used as an independent product, the integrated unit may be stored in a computer-readable storage medium. Based on such an understanding, in this application, all or some of the procedures of the method in the foregoing embodiment may be implemented by a computer program instructing related hardware. The computer program may be stored in a computer-readable storage medium. When the computer program is executed by a processor, the steps in the foregoing method embodiment can be implemented. The computer program includes computer program code, and the computer program code may be in a source code form, an object code form, an executable file form, some intermediate forms, or the like. The computer-readable medium may include any entity or apparatus that can carry the computer program code, a recording medium, a USB flash drive, a removable hard disk, a magnetic disk, a compact disc, a computer memory, a read-only memory (ROM, Read-Only Memory), a random access memory (RAM, Random Access Memory), an electrical carrier signal, a telecommunication signal, a software distribution media, and the like. It should be noted that content included in the computer-readable medium may be appropriately added or deleted based on requirements of legislation and patent practice in a jurisdiction. For example, in some jurisdictions, according to legislation and patent practice, the computer-readable medium does not include the electrical carrier signal or the telecommunication signal.

The foregoing embodiments are merely intended to describe the technical solutions of this application, but not to limit this application. The invention is defined by the appended claims.

## Claims

1. A heart status assessment apparatus, comprising:
a sample obtaining module (601), configured to obtain heart rhythm sample data, wherein the heart rhythm sample data comprises sample heart rhythm features and sample labels;
a model training module (602), configured to train a preset scoring model based on the heart rhythm sample data, to obtain a heart rhythm scoring model; and
a status assessment module (603), configured to input a to-be-recognized heart rhythm feature into the heart rhythm scoring model, to obtain a heart status assessment score, **characterised in that**
ranks of different arrhythmia events are used as the sample labels, and the preset scoring model is a ranking model.

2. The heart status assessment apparatus according to claim 1, wherein the apparatus further comprises:
a scaling selection module, configured to determine a scaling algorithm based on the sample labels and a preset interval; and
correspondingly, the status assessment module (603) comprises:
a first assessment submodule, configured to input the to-be-recognized heart rhythm feature into the heart rhythm scoring model, to obtain a first assessment score;
a second assessment submodule, configured to calculate the first assessment score by using the scaling algorithm, to obtain a second assessment score; and
a score assessment submodule, configured to use the second assessment score as the heart status assessment score.

3. The heart status assessment apparatus according to claim 2, wherein the score assessment submodule comprises:
an in-interval submodule, configured to use the second assessment score as the heart status assessment score if the second assessment score is within the preset interval;
a lower limit submodule, configured to use a lower limit as the heart status assessment score if the second assessment score is less than the lower limit of the preset interval; and
an upper limit submodule, configured to use an upper limit as the heart status assessment score if the second assessment score is greater than the upper limit of the preset interval.

4. The heart status assessment apparatus according to claim 1, wherein the apparatus further comprises:
a score obtaining module, configured to obtain all heart status assessment scores that are within a preset time period; and
a first prediction module, configured to match all the heart status assessment scores that are within the preset time period with heart score curves in a preset database, wherein a heart score curve with a highest matching degree is presented as a prediction curve.

5. The heart status assessment apparatus according to any one of claims 1 to 4, wherein the sample obtaining module (601) comprises:
a ranking label submodule, configured to rank all the pieces of heart rhythm sample data based on a sample heart rhythm feature of each piece of heart rhythm sample data and a preset ranking rule, to obtain ranks of all the pieces of heart rhythm sample data.

6. The heart status assessment apparatus according to any one of claims 1 to 5, wherein the sample obtaining module (601) comprises:
a window-based processing submodule, configured to obtain original data and perform window-based processing on the original data based on a preset time window, to obtain at least one data segment; and
a feature extraction submodule, configured to extract a heart rhythm feature of the data segment, to obtain a sample heart rhythm feature.

7. The heart status assessment apparatus according to any one of claims 1 to 6, wherein the apparatus further comprises:
a score obtaining module, configured to obtain all heart status assessment scores that are within a preset time period; and
a second prediction module, configured to perform curve fitting on all the heart status assessment scores that are within the preset time period, to obtain and present a prediction curve.

8. The heart status assessment apparatus according to any one of claims 1 to 7, wherein the apparatus further comprises:
a prompt operation module, configured to perform, based on a prompt condition that the heart status assessment score meets, a preset prompt operation corresponding to the prompt condition.

9. A terminal device implementing the heart status assessment apparatus according to any one of claims 1 to 8.

## Patentansprüche

1. Einrichtung für eine Beurteilung eines Herzzustands, die umfasst:
ein Probenerhaltungsmodul (601), das konfiguriert ist, um Herzrhythmusprobendaten zu erhalten, wobei die Herzrhythmusprobendaten Probenherzrhythmusmerkmale und Probenbezeichnungen umfassen;
ein Modelltrainingsmodul (602), das konfiguriert ist, um ein voreingestelltes Bewertungsmodell basierend auf den Herzrhythmusprobendaten zu trainieren, um ein Herzrhythmusbewertungsmodell zu erhalten; und
ein Zustandsbeurteilungsmodul (603), das konfiguriert ist, um ein zu erkennendes Herzrhythmusmerkmal in das Herzrhythmusbewertungsmodell einzugeben, um einen Beurteilungswert des Herzzustands zu erhalten, **dadurch gekennzeichnet, dass** Ränge unterschiedlicher Arrhythmieereignisse als Probenbezeichnungen verwendet werden und das voreingestellte Bewertungsmodell ein Rangfolgemodell ist.

2. Einrichtung für die Beurteilung des Herzzustands nach Anspruch 1, wobei die Einrichtung ferner umfasst:
ein Skalierungsauswahlmodul, das konfiguriert ist, um einen Skalierungsalgorithmus basierend auf den Probenbezeichnungen und einem voreingestellten Intervall zu bestimmen; und
entsprechend, das Zustandsbeurteilungsmodul (603) umfasst:
ein erstes Beurteilungsuntermodul, das konfiguriert ist, um das zu erkennende Herzrhythmusmerkmal in das Herzrhythmusbewertungsmodell einzugeben, um einen ersten Beurteilungswert zu erhalten;
ein zweites Beurteilungsuntermodul, das konfiguriert ist, um den ersten Beurteilungswert durch Verwenden des Skalierungsalgorithmus zu berechnen, um einen zweiten Beurteilungswert zu erhalten; und
ein Wertebeurteilungsuntermodul, das konfiguriert ist, um den zweiten Beurteilungswert als den Beurteilungswert des Herzzustands zu verwenden.

3. Einrichtung für die Beurteilung des Herzzustands nach Anspruch 2, wobei das Wertebeurteilungsuntermodul umfasst:
ein In-Intervall-Untermodul, das konfiguriert ist, um den zweiten Beurteilungswert als den Beurteilungswert des Herzzustands zu verwenden, falls der zweite Beurteilungswert innerhalb des voreingestellten Intervalls liegt;
ein Untergrenzenuntermodul, das konfiguriert ist, um eine Untergrenze als den Beurteilungswert des Herzzustands zu verwenden, falls der zweite Beurteilungswert weniger als die Untergrenze des voreingestellten Intervalls ist; und
ein Obergrenzenuntermodul, das konfiguriert ist, um eine Obergrenze als den Beurteilungswert des Herzzustands zu verwenden, falls der zweite Beurteilungswert höher als die Obergrenze des voreingestellten Intervalls ist.

4. Einrichtung für die Beurteilung des Herzzustands nach Anspruch 1, wobei die Einrichtung ferner umfasst:
ein Werterhaltungsmodul, das konfiguriert ist, um alle Beurteilungswerte der Herzzustands, die innerhalb eines voreingestellten Zeitraums liegen, zu erhalten; und
ein erstes Vorhersagemodul, das konfiguriert ist, um alle Beurteilungswerte des Herzzustands, die innerhalb des voreingestellten Zeitraums liegen, mit Herzwertkurven in einer voreingestellten Datenbank abzugleichen, wobei eine Herzwertkurve mit einem höchsten Übereinstimmungsgrad als eine Vorhersagekurve dargestellt wird.

5. Einrichtung für die Beurteilung des Herzzustands nach einem der Ansprüche 1 bis 4, wobei das Probenerhaltungsmodul (601) umfasst:
ein Rangfolgebezeichnungsuntermodul, das konfiguriert ist, um alle der Teile der Herzrhythmusprobendaten basierend auf einem Probenherzrhythmusmerkmal jedes Teils der Herzrhythmusprobendaten und einer voreingestellten Rangfolgeregel zu rangieren, um Ränge aller der Teile der Herzrhythmusprobendaten zu erhalten.

6. Einrichtung für die Beurteilung des Herzzustands nach einem der Ansprüche 1 bis 5, wobei das Probenerhaltungsmodul (601) umfasst:
ein fensterbasiertes Verarbeitungsuntermodul, das konfiguriert ist, um Originaldaten zu erhalten und ein fensterbasiertes Verarbeiten auf den Originaldaten basierend auf einem voreingestellten Zeitfenster durchzuführen, um mindestens ein Datensegment zu erhalten; und
ein Merkmalsextraktionsuntermodul, das konfiguriert ist, um ein Herzrhythmusmerkmal des Datensegments zu extrahieren, um ein Probenherzrhythmusmerkmal zu erhalten.

7. Einrichtung für die Beurteilung des Herzzustands nach einem der Ansprüche 1 bis 6, wobei die Einrichtung ferner umfasst:
ein Werterhaltungsmodul, das konfiguriert ist, um alle Beurteilungswerte der Herzzustands, die innerhalb eines voreingestellten Zeitraums liegen, zu erhalten; und
ein zweites Vorhersagemodul, das konfiguriert ist, um eine Kurvenanpassung an allen der Beurteilungswerte des Herzzustands, die innerhalb des voreingestellten Zeitraums liegen, durchzuführen, um eine Vorhersagekurve zu erhalten und darzustellen.

8. Einrichtung für die Beurteilung des Herzzustands nach einem der Ansprüche 1 bis 7, wobei die Einrichtung ferner umfasst:
ein Promptoperationsmodul, das konfiguriert ist, um, basierend auf einer Promptbedingung, die der Beurteilungswert des Herzzustands erfüllt, eine voreingestellte Promptoperation, die der Promptbedingung entspricht, durchzuführen.

9. Endgerät, das die Einrichtung für die Beurteilung des Herzzustands nach einem der Ansprüche 1 bis 8 implementiert.

## Revendications

1. Appareil d'évaluation d'état de cœur comprenant :
un module d'obtention d'échantillons (601), configuré pour obtenir des données d'échantillons de rythme cardiaque, dans lequel les données d'échantillons de rythme cardiaque comprennent des caractéristiques d'échantillons de rythme cardiaque et des étiquettes d'échantillons ;
un module d'entraînement de modèle (602), configuré pour entraîner un modèle de notation prédéfini sur la base des données d'échantillons de rythme cardiaque, afin d'obtenir un modèle de notation de rythme cardiaque ; et
un module d'évaluation d'état (603), configuré pour entrer une caractéristique de rythme cardiaque à reconnaître dans le modèle de notation de rythme cardiaque, afin d'obtenir un score d'évaluation d'état de cœur, **caractérisé en ce que** des rangs de différents événements d'arythmie sont utilisés comme étiquettes d'échantillon, et le modèle de notation prédéfini est un modèle de classement.

2. Appareil d'évaluation d'état de cœur selon la revendication 1, dans lequel l'appareil comprend en outre :
un module de sélection d'échelle, configuré pour déterminer un algorithme de mise à l'échelle sur la base des étiquettes d'échantillons et d'un intervalle prédéfini ; et
en conséquence, le module d'évaluation d'état (603) comprend :
un premier sous-module d'évaluation, configuré pour entrer la caractéristique de rythme cardiaque à reconnaître dans le modèle de notation de rythme cardiaque, afin d'obtenir un premier score d'évaluation ;
un second sous-module d'évaluation, configuré pour calculer le premier score d'évaluation à l'aide de l'algorithme de mise à l'échelle, afin d'obtenir un second score d'évaluation ; et
un sous-module d'évaluation des scores, configuré pour utiliser le second score d'évaluation comme score d'évaluation d'état de cœur.

3. Appareil d'évaluation d'état de cœur selon la revendication 2, dans lequel le sous-module d'évaluation de score comprend :
un sous-module d'intervalle, configuré pour utiliser le second score d'évaluation comme score d'évaluation d'état de cœur, si le second score d'évaluation se situe dans l'intervalle prédéfini ;
un sous-module de limite inférieure, configuré pour utiliser une limite inférieure comme score d'évaluation d'état de cœur, si le second score d'évaluation est inférieur à la limite inférieure de l'intervalle prédéfini ; et
un sous-module de limite supérieure, configuré pour utiliser une limite supérieure comme score d'évaluation d'état de cœur, si le second score d'évaluation est supérieur à la limite supérieure de l'intervalle prédéfini.

4. Appareil d'évaluation d'état de cœur selon la revendication 1, dans lequel l'appareil comprend en outre :
un module d'obtention de scores, configuré pour obtenir tous les scores d'évaluation d'état de cœur qui se situent dans une période de temps prédéfinie ; et
un premier module de prédiction, configuré pour faire correspondre tous les scores d'évaluation d'état de cœur qui se situent dans la période prédéfinie avec des courbes de score cardiaque d'une base de données prédéfinie, dans lequel une courbe de score cardiaque présentant un degré de correspondance le plus élevé est présentée comme une courbe de prédiction.

5. Appareil d'évaluation d'état de cœur selon l'une quelconque des revendications 1 à 4, dans lequel le module d'obtention d'échantillons (601) comprend :
un sous-module de classement d'étiquettes, configuré pour classer toutes les données d'échantillons de rythme cardiaque sur la base d'une caractéristique d'échantillons de rythme cardiaque de chaque donnée d'échantillons de rythme cardiaque et d'une règle de classement prédéfinie, afin d'obtenir des classements de toutes les données d'échantillons de rythme cardiaque.

6. Appareil d'évaluation d'état de cœur selon l'une quelconque des revendications 1 à 5, dans lequel le module d'obtention d'échantillons (601) comprend :
un sous-module de traitement par fenêtre, configuré pour obtenir des données originales et effectuer un traitement par fenêtre sur les données originales sur la base d'une fenêtre temporelle prédéfinie, afin d'obtenir au moins un segment de données ; et
un sous-module d'extraction de caractéristiques, configuré pour extraire une caractéristique de rythme cardiaque du segment de données, afin d'obtenir une caractéristique de rythme cardiaque d'échantillon.

7. Appareil d'évaluation d'état de cœur selon l'une quelconque des revendications 1 à 6, dans lequel l'appareil comprend en outre :
un module d'obtention de scores, configuré pour obtenir tous les scores d'évaluation d'état de cœur qui se situent dans une période de temps prédéfinie ; et
un second module de prédiction, configuré pour effectuer un ajustement de courbe sur tous les scores d'évaluation d'état de cœur qui se situent dans la période prédéfinie, afin d'obtenir et de présenter une courbe de prédiction.

8. Appareil d'évaluation d'état de cœur selon l'une quelconque des revendications 1 à 7, dans lequel l'appareil comprend en outre :
un module d'opération rapide, configuré pour effectuer, sur la base d'une condition rapide que le score d'évaluation d'état de cœur remplit, une opération rapide prédéfinie correspondant à la condition rapide.

9. Dispositif terminal mettant en œuvre l'appareil d'évaluation d'état de cœur selon l'une quelconque des revendications 1 à 8.
